Europäisches Patentamt

European Patent Office

Office européen des brevets

(11) Publication number: **0 227 955**
**A2**

## (12) EUROPEAN PATENT APPLICATION

(21) Application number: 86116467.1

(51) Int. Cl.⁴: **A61L 15/01** , A61F 13/00

(22) Date of filing: 20.07.83

(30) Priority: 21.07.82 GB 8221044

(43) Date of publication of application:
08.07.87 Bulletin 87/28

(60) Publication number of the earlier application in accordance with Art.76 EPC: 0 099 758

(84) Designated Contracting States:
AT BE CH DE FR GB IT LI LU NL SE

(71) Applicant: **University of Strathclyde**
**McCance Building 16 Richmond Street**
**Glasgow G1 1YQ, Scotland(GB)**

(72) Inventor: **Juhasz, Laszlo**
**51 Kilmardinny Avenue**
**Bearsden Glasgow G61 3NL Scotland(GB)**

(74) Representative: **Wotherspoon, Graham et al**
**FITZPATRICKS 4 West Regent Street**
**Glasgow G2 1RS Scotland(GB)**

(54) Composite wound dressing.

(57) The multilayered composite wound dressing comprises of a semipermeable membrane (10), optionally with a permeable supporting and reinforcing layer, and a non-stick, self-sealing biodegradable tissue interface (11). The semipermeable membrane may be a synthetic collagen, an alginate or a biocompatible polymer and it controls the rate of water vapour transmission from the wound surface. The permeable supporting and reinforcing layer provides mechanical strength and is usually a textile fabric. The biodegradable tissue interface may be synthetic collagen, sodium-calcium alginate or collagen-alginate complex, and it provides non-stick haemostatic sealing and aids the wound repair processes. For topical medication, growth promoting, antibacterial, antiallergic and therapeutic agents may also be incorporated.

FIG.1

EP 0 227 955 A2

## COMPOSITE WOUND DRESSING

The present invention relates to composite wound dressings particularly, but not exclusively, for use as a general surgical wound dressing, burn dressing, donor site dressing, bedsore dressing, infected ulcer dressing and like applications. In order for a composite wound dressing to be as effective and efficient as normal skin, it must simulate the function of normal skin. There are three main requirements that normal skin fulfils; firstly it provides controlled water vapour permeability, secondly the skin provides a tough mechanical barrier between the tissues and the external environment with uniform pressure distribution and acts as a barrier to infection and thirdly it provides a bioactive tissue interface to maintain an adaptive physiological performance and intracellular/extracellular activities in wound repair. It is therefore considered that a composite wound dressing should satisfy all of these basic requirements and take part in the fundamental activities of the wound healing process. The author considers that, at the present time, there is no composite wound dressing available which satisfies all these requirements.

It is an object of the present invention to obviate or to mitigate the disadvantages associated with current composite wound dressings.

According to the present invention there is provided a composite wound dressing comprising in a layered arrangement, a semipermeable membrane and a biodegradable tissue interface, said semipermeable membrane is formed from a synthetic collagen, an alginate or a biocompatible polymer selected from polyurethane, polypropylene and silicone rubber, and in that the biodegradable tissue interface is formed from a synthetic collagen, a Na/Ca alginate or a collagen/ alginate complex.

Said dressing may include a supporting and reinforcing permeable layer, said permeable layer being located between the semipermeable membrane and the biodegradable tissue interface.

Said biodegradable interface may be a synthetic collagen produced from animal sources such as calf skin and intestines. After several stages of purification, a collagen film or fabric is prepared for use. Alternatively, the biodegradable tissue interface is a Na-Ca alginate produced from seaweeds. Preferably, the said biodegradable tissue interface is a collagen-alginate complex with a collagen/alginate ratio of 0.1 -30, and it is prepared as a porous structure.

Preferably also, the semipermeable membrane is a synthetic collagen. Alternatively, the semipermeable membrane may be an alginate or a biocompatible polymer such as polyurethane, polypropylene or silicone rubber, etc.

An embodiment of the present invention will now be described by way of example with reference to the accompanying drawings, in which:-

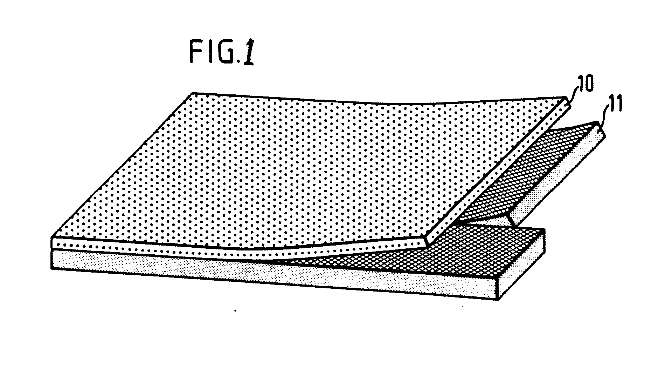

Fig 1 is a schematic diagram of a composite wound dressing for use of a general wound dressing.

Referring now to Fig 1 of the drawings, the composite wound dressing comprises a polyurethane polymer 10 as the semipermeable membrane and the biodegradable tissue interface is a collagen-alginate or (Na-Ca) alginate 11. This dressing is particularly suitable for use as a general surgical wound dressing.

The biodegradable alginate is produced from brown seaweed (phaeophycae) which contains naturally occurring polysaccharides. The BdA is prepared by purifying and acidifying to a various degree the sodium alginate to obtain a slow dissolving material. In principle, therefore BdA contains both sodium and calcium alginate with a ratio of Na/Ca = 0.1 to 50.

The dressing is placed on a wound, for example a burn with the biodegradable alginate in contact with the wound surface. Once the BdA 3 is in contact with tissue fluid, ie such as exudate, it dissolves at a predetermined rate. In this hydrolysed state, it produces a viscous polyelectrolyte film which provides haemostatic wound sealing an electrical charge transfer of the sodium and calcium ions from an external source. The multilayered design is similar in structure to normal skin and provides near physiological performance since the semipermeable membrane controls the rate of water vapour transmission from the tissue. The supporting fabric provides increased mechanical strength with uniform pressure and electrical field distribution and the biodegradable tissue interface 3 provides haemostatic sealing.

The protein concentration in the blood vessels can be maintained at a high level and consequently water will be drawn back into these vessels by osmosis. This reestablishment of osmotic feedback prevents the accumulation of both water and proteins in the surrounding tissue, thus reducing local pain and inflammation due to the fluid pressure on the sensory nerve endings.

Without departing from the scope of the invention, it will be understood that different materials may be used in the manufacture of the composite wound dressing according to the invention. For example, the semipermeable membrane may be made of biodegradable materials such as a synthetic collagen, alginate or other biodegradable polymer or a biocompatible material such as polyurethane, polypropylene, silicone or natural rubber. The supporting and reinforcing fabric may be coated, impregnated or plated with materials such as silver, zinc, gold, platinum or carbon.

It is also possible to omit the supporting and reinforcing layer should the semipermeable membrane and the biodegradable tissue interface be made sufficiently strong to facilitate handling.

In addition, topical growth promoting, antibacterial or antiallergic agents such as silver sulphadiazene, zinc and other substances may be incorporated into the dressing, preferably into the collagen, alginate or collagen-alginate of the biodegradable tissue interface.

The advantages of the composite wound dressing according to the present invention include; control of the rate of water vapour transmission from the wound thereby presenting local dehydration, all processes in the wound healing phase are enhanced, eg inflammation is reduced, epithelialisation and collagen synthesis is increased, its layered structure gives it flexibility and mechanical strength and facilitates easy handling, and the dressing is self-sealing non-tissue adhesive, simply peeling off when required, and reduced scar formation and improved clinical appearance are an advantage of using this composite wound dressing.

Claims

1. A composite wound dressing comprising in a layered arrangement, a semipermeable membrane and a non-stick, self-sealing biodegradable tissue interface, characterised in that said semipermeable membrane is formed from a synthetic collagen, an alginate or a biocompatible polymer selected from polyurethane, polypropylene and silicone rubber, and in that the biodegradable tissue interface is formed from a synthetic collagen, a Na/Ca alginate or a collagen/alginate complex.

2. A composite wound dressing according to claim 1 wherein a supporting and reinforcing permeable layer is located between the semipermeable membrane and the biodegradable tissue interface.

3. A composite wound dressing according to claim 1 or claim 2 wherein the said tissue interface is formed from synthetic collagen produced from animal sources.

4. A composite wound dressing according to claim 1 or claim 2 wherein the said tissue interface is formed from a sodium/calcium alginate mixture with a Na/Ca ratio of 0.1 to 50 which is derived from seaweed.

5. A composite wound dressing according to claim 1 or claim 2 wherein the said tissue interface is formed from a collagen/alginate complex with a collagen/alginate ratio of 0.1 to 30 and is produced as a semipermeable film or an opened foamed structure.

6. A composite wound dressing according to any one of the preceding claims wherein the semipermeable membrane is a synthetic collagen.

7. A composite wound dressing according to any one of claims 1 to 5 wherein the semipermeable membrane is an alginate.

FIG.1